# EUROPEAN PATENT APPLICATION

(11) **EP 2 433 726 A1**
(43) Date of publication of application: **28.03.2012**
(21) Application number: 11250814.8
(22) Date of filing: 20.09.2011
(51) Int. Cl.: B22F 1/00, B22F 9/24, A61K 49/00, A61K 49/04, A61K 49/18

(54) **CT/MRI dual-modality molecular imaging contrast agent and method for manufacturing the same**

(30) Priority: 28.09.2010 TW 99132819
(71) Applicant: National Cheng Kung University, Tainan City 701 (TW); National Taiwan Normal University, Da'an Dist . Taipei City 106 (TW)
(72) Inventor: Shieh, Dar-Bin, Tainan City 704 (TW); Chen, Chia-Chun, Taipei City 112 (TW); Chou, Shang-Wei, Hualien County 973 (TW); Wu, Ping-Ching, Tainan City 701 (TW); Hsiao, Yu-Hong, Taichung City 436 (TW); Yang, Yu-Sang, Hsinchu City 300 (TW)
(74) Representative: Matthews, Derek Peter

(57) **Abstract**

The present invention relates to a CT/MRI dual modality molecular imaging contrast agent and a method for manufacturing the same. In the present invention, the CT/MRI dual modality molecular imaging contrast agent comprises: an FePt nanoparticle; and a surface modification molecular binding to a surface of the FePt nanoparticle to modify the FePt nanoparticle with an amino-group, a carboxyl-group, or a biotin, wherein the FePt nanoparticle is in a form of sphere, octapod, cuboctahedron, cube, or truncated cube, and the FePt nanoparticle is represented by the following formula (I):

FeₓPt₁₀₀₋ₓ (I)

wherein, x is 30∼60.

## Description

### CROSS REFERENCE TO RELATED APPLICATION

This application claims the benefits of the Taiwan Patent Application Serial Number 99132819, filed on September 28, 2010, the subject matter of which is incorporated herein by reference.

### BACKGROUND OF THE INVENTION

### 1. Field of the Invention

The present invention relates to a multi-modality imaging contrast agent and a method for manufacturing the same and, more particularly, to a computerized tomography/magnetic resonance imaging dual-modality molecular imaging contrast agent and a method for manufacturing the same.

### 2. Description of Related Art

Imaging examinations are general clinical methods used in diagnostics and therapeutics. During performing the imaging examinations, imaging contrast agents are generally used to enhance the contrast between different tissues, in order to improve the visibility of tissues to be examined. In recent years, the common imaging examinations used in clinic comprises computerized tomography (CT) and magnetic resonance imaging (MRI).

Computed tomography is a medical imaging method for producing two-dimensional (2-D) and three-dimensional (3-D) cross-sectional images of an object from flat X-ray images. After a series of 2-D X-ray images taken around a single axis of rotation, a digital geometry processing is used to generate a 3-D image of the inside of an image. In addition, the computed tomography gives high-resolution tomography information and shows the whole symptoms, so it is one of the most routinely applied medical imaging modality in clinic.

Iodinated molecules and compounds with high X-ray absorption coefficient have been used for CT contrast enhancement for decades, wherein iodinated molecules are ionic imaging contrast agents with high permeability. When the iodinated molecules are used as imaging contrast agents, it has disadvantages of renal toxicity and short imaging times due to rapid clearance by the kidney. Hence, some nanoparticle-based imaging contrast agents such as polymer-coated bismuth sulfide (Bi₂S₃) and polyethylene glycol (PEG)-coated Au nanoparticles are developed, in order to overcome the disadvantages of the iodinated molecules.

On the other hand, magnetic resonance imaging (MRI) is a medical imaging method to visualize detailed internal structures of an object. During the examination, the MRI machine provides a powerful magnetic field to align the magnetization of hydrogen atoms in the body, and then a section image of an organ of the object can be obtained after digital processing. The MRI technique has advantages of non-ionizing radiation, and high sensitivity to the distribution of water and other biomolecules, so it has been applied in clinic for the diagnosis of many diseases. Furthermore, MRI has good contrast between different soft tissues of the body, so it does not need any imaging contract agents for most of the MRI examination. However, some imaging contrast agents still have to be applied to the body when fine MRI examination is to be performed. Two major types of MRI image contrast agents are conventionally used in clinic, which comprises: gadolinium (Gd³⁺)-based T₁ contrast agents and superparamagnetic iron oxide nanoparticle-based T₂ contrast agents.

CT and MRI examinations are generally used for diagnostics and therapeutics. However, when CT and MRI examinations are performed on a patient, the second examination cannot be performed until the imaging contrast agent of the first examination is totally eliminated from the patient. Hence, the time for performing imaging examinations on one patient is increased. Recently, few imaging contrast agents with CT/MRI dual imaging contrast effect has been developed, such as Gd chelated Au nanoparticles. However, the process for preparing this dual imaging contrast agent is very complicated, and the imaging contrast effect thereof still has to be improved.

Therefore, it is desirable to provide a CT/MRI dual modality imaging contrast agent, which is prepared in a simple process and has good imaging contrast effect, to reduce the time for examination in clinic.

### SUMMARY OF THE INVENTION

The object of the present invention is to provide a method for manufacturing a CT/MRI dual-modality imaging contrast agent, which can be used to prepare a dual-modality imaging contrast agent for CT and MRI examination in a simple process.

Another object of the present invention is to provide a CT/MRI dual-modality imaging contrast agent. When the imaging contrast agent of the present invention is applied to the CT and/or MRI examination, the problem of renal toxicity and other uncomfortable symptoms can be solved.

To achieve the object, the method for manufacturing a CT/MRI dual-modality imaging contrast agent comprises the following steps: (A) providing a mixture solution containing a Pt compound, a Fe compound, a polyol, a surfactant, and a solvent; (B) heating the mixture solution to react the Pt compound with the Fe compound to obtain FePt nanoparticles; (C) cooling the mixture solution, and separating the FePt nanoparticles from the mixture solution; and (D) mixing the FePt nanoparticles with a surface modification molecule to obtain surface-modified FePt nanoparticles. Herein, the surface-modified FePt nanoparticles are CT/MRI dual-modality imaging contrast agents.

In addition, the present invention further provides a CT/MRI dual-modality imaging contrast agent prepared through the aforementioned process, which comprises: an FePt nanoparticle; and a surface modification molecule binding to a surface of the FePt nanoparticle. Herein, the surface modification molecule contains an amino-group, a carboxyl-group, or a biotin, so the surface of the FePt nanoparticle is modifid with the amino-group, the carboxyl-group, or the biotin. In addition, the FePt nanoparticle is in a form of sphere, octapod, cuboctahedron, cube, or truncated cube. Furthermore, the FePt nanoparticle is represented by the following formula (I):

FeₓPt₁₀₀₋ₓ (I)

wherein, x is 30-60.

According to the CT/MRI dual-modality imaging contrast agent and the method for manufacturing the same of the present invention, the nanoparticles with high biocompatibility can be prepared through a simple process. In addition, the surfaces of the FePt nanoparticles are modified with surface modification molecules, and the surface-modified functional groups are exposed to connect to biomolecules. Furthermore, the imaging contrast agent of the present invention can be used as not only a CT imaging contrast agent, but also a MRI contrast agent. In addition, the dual-modality imaging contrast agent of the present invention can further improve the imaging contrast effect of the CT and MRI examination. Compared to the conventional imaging contrast agent, the dual-modality imaging contrast agent of the present invention has high safety, and low rental toxicity.

According to the CT/MRI dual-modality imaging contrast agent and the method for manufacturing the same of the present invention, the Pt compound can be at least one selected from the group consisting of Pt(acac)₂, PtCl₂, PtCl₄, H₂PtCl₄, H₂PtCl₆, K₂PtCl₄, and K₂PtCl₆. In addition, the Fe compound can be at least one selected from the group consisting of Fe(CO)₅, Fe(acac)₂, Fe(acac)₃, and Fe-oleate. Furthermore, the polyol can be any C₈₋₂₀ alcohol. Preferably, the polyol is at least one selected from the group consisting of 1,2-hexa-decanediol, 1,2-Dodecanediol, and polyethylene glycol.

In addition, the surface modification molecules used in the present invention can be amino-group modification molecules containing amino groups, such as cysteinamine, cystamine, or mercaptoalkylamine. Preferably, the amino-group modification molecules have mercapto groups (-SH) and amino groups (-NH₂). In the present invention, the amino-group modification molecules bind to the surfaces of FePt nanoparticles through the mercapto groups to obtain the FePt nanoparticles modified with the amino groups. In addition, the surfaces of the FePt nanoparticles can be modified with not only the amino-group modification molecules, but also functional groups capable of conjugating with biomolecules, such as molecules with carboxyl groups (-COOH), biotin, and nitrilotriacetate.

According to the CT/MRI dual-modality imaging contrast agent and the method for manufacturing the same of the present invention, the surfactant can be oleic acid, oleyl amine, or a combination thereof. Preferably, the surfactant comprises oleic acid, and oleyl amine. More preferably, the volume ratio between the oleic acid and the oleyl amine is 1:1 to 3:1. In the present invention, the shapes and the particle sizes of the FePt nanoparticles can be adjusted by controlling the volume ratio between the components of the surfactant. In addition, the diameter of the FePt nanoparticles may be 1-100 nm. Preferably, the diameter of the FePt nanoparticles is 1-20 nm. The *in vivo* distribution, the pharmacokinetics, the metabolism, and the clearance time of the FePt nanoparticles depends upon the shapes and the particle sizes thereof.

When the volume ratio between the oleic acid and the oleyl amine is 1:1, the obtained FePt nanoparticles represent the lattice spacing of (111) facet of octapod structure. When the volume ratio between the oleic acid and the oleyl amine is 3:1, the obtained FePt nanoparticles represent the lattice spacing of (111) and (100) facets of cuboctahedron structure. When the reaction time is reduced, the obtained FePt nanoparticles is in a form of sphere.

The method of manufacturing the CT/MRI dual-modality imaging contrast agent of the present invention can further comprise a step (E) after the step (D): mixing the surface-modified FePt nanoparticles with target molecules to obtain a dual-modality targeting imaging contrast agent. Hence, the CT/MRI dual-modality imaging contrast agent of the present invention may further comprise target molecules binding to the surface modification molecules, wherein the target molecules can be antibodies, proteins, nucleic acids, nano-objects, or molecular templates. Preferably, the target molecules are antibodies, which can be selected according to tissues or organs to be detected. For example, the target molecules are anti-Her2 antibodies, wherein the Her2 is one kind of human epidermal growth factor receptor. When amino-group modification molecules bind to the surfaces of FePt nanoparticles, the surfaces of the FePt nanoparticles are modified with amino groups (-NH₂). In this case, molecules with carboxyl groups (-COOH) can be used as the target molecules, which can bind to the amino-group modification molecules through the interaction between the amino groups and the carboxyl groups, to obtain dual-modality targeting imaging contrast agents. Hence, according to the CT/MRI dual-modality imaging contrast agent and the method for manufacturing the same of the present invention, the surfaces of the FePt nanoparticles can be properly biologically modified through a chemical modification process. After the FePt nanoparticles are modified with the target molecules, the obtained dual-modality targeting imaging contrast agents can precisely target to the position of tumors in an object.

Other objects, advantages, and novel features of the invention will become more apparent from the following detailed description when taken in conjunction with the accompanying drawings.

### BRIEF DESCRIPTION OF THE DRAWINGS

FIG. 1 is a perspective view of a CT/MRI dual-modality imaging contrast agent of Embodiment 4 of the present invention;
FIG. 2 is a hysteresis loop of CT/MRI dual-modality imaging contrast agents of Embodiments 1-3 of the present invention;
FIG. 3 is a diagram showing the results of MTT assay of CT/MRI dual-modality imaging contrast agents of Embodiments 1-3 of the present invention;
FIG. 4 is a diagram showing the results of hemolysis test of CT/MRI dual-modality imaging contrast agents of Embodiments 1-3 of the present invention;
FIG. 5 is a diagram showing the results of biodistribution of CT/MRI dual-modality imaging contrast agents of Embodiments 1-3 of the present invention;
FIG. 6 is a diagram showing the results of *in vitro* MRI examination of CT/MRI dual-modality imaging contrast agents of Embodiments 1-3 of the present invention;
FIG. 7 is a diagram showing the results of *in vitro* CT examination of CT/MRI dual-modality imaging contrast agents of Embodiments 1-3 of the present invention;
FIG. 8 is a diagram showing the results of *in vitro* MRI examination of CT/MRI dual-modality imaging contrast agents of Embodiments 4 and 6 of the present invention; and
FIG. 9 is a diagram showing the results of *in vitro* CT examination of CT/MRI dual-modality imaging contrast agents of Embodiments 4 and 6 of the present invention.

### DETAILED DESCRIPTION OF THE PREFERRED EMBODIMENT

The present invention has been described in an illustrative manner, and it is to be understood that the terminology used is intended to be in the nature of description rather than of limitation. Many modifications and variations of the present invention are possible in light of the above teachings. Therefore, it is to be understood that within the scope of the appended claims, the invention may be practiced otherwise than as specifically described.

In the present invention, the material used for manufacturing CT/MRI dual-modality imaging contrast agents are shown as follow: Platinum acetylacetonate (Pt(acac)₂, ACROS, 97%), iron pentacarbonyl (Fe(CO)₅, Aldrich, 99.99%), 1,2-hexadecanediol (Aldrich, 90%), dioctyl ether (ACROS, 90%), oleyl amine (Aldirch, 70%), oleic acid (Aldrich, 90%), and cysteinamine (Cyam, Sigma, 95%).

### Embodiment 1 - Preparation of 3-4 nm FePt nanoparticles

Pt(acac)₂ (97 mg), 1,2-hexadecandiol (195 mg) and dioctyl ether (10 mL) were mixed and heated to 100°C under N₂ for 10 min. Fe(CO)₅ (66 µL), oleyl amine (80 µL) and oleic acid (80 µL) were injected at 100 °C. The reaction mixture was heated to 297 °C. After 30 min, the heating source was removed and the product was cooled to the room temperature. The product was precipitated by adding ethanol and separated by centrifugation. After the aforementioned process, FePt nanoparticles with the lattice spacing of (111) were obtained.

Herein, Transmission electron microscopy (TEM) and Energy-dispersive X-ray spectroscopy (EDX) were carried out on a JEOL Philips/FEI Tecnai 20 G2 S-Twin transmission electron microscope to characterize the properties of the FePt nanoparticles of the present invention. In addition, the FePt nanoparticles prepared in the following Embodiments 2 and 3 were also characterized by the same method described in the present embodiment. Hence, the descriptions about the characterization of the properties of the FePt nanoparticles of the following embodiments are omitted in the present specification.

First, a small amount of FePt nanoparticles was deposited in toluene. A drop of solution fell on an amorphous carbon membrane supported by a copper grid. The FePt nanoparticles were determined at magnification of 50k, 100k and 200k. Powder x-ray diffraction data was collected on a Bruker D8 Advance diffractometer. In addition, the powder of the FePt nanoparticles was also placed on amorphous Si wafer and the workup procedure was carried out with Cu Kα radiation (λ = 1.54178 A). Magnetic measurements were performed by superconducting quantum interference device (SQUID) magnetometer (MPMS, Quantum Design). The measurements were recorded between -10000 Oe and 10000 Oe at 300 K.

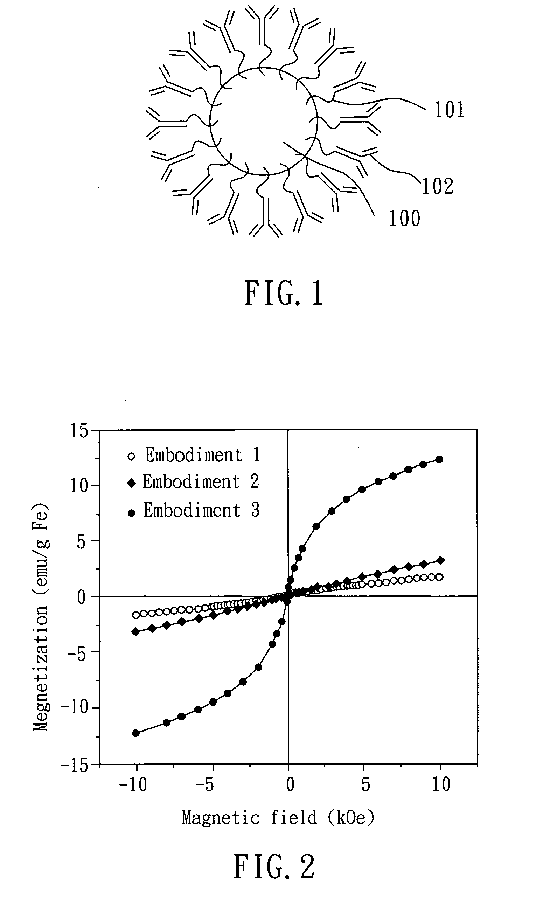

The TEM image shows that the FePt nanoparticles of the present embodiment have particle sizes of 3.58±0.34 nm. In addition, the EDX analysis revealed that the composition of the FePt nanoparticles of the present embodiment is Fe₅₈Pt₄₂. Furthermore, the result of the magnetic measurement shows that the saturated mass magnetization (Mₛ) of the FePt nanoparticles of the present embodiment at 300 K is 1.7 emu/g Fe, as shown in FIG. 2.

### Embodiment 2 - Preparation of 6-7 nm FePt nanoparticles

Pt(acac)₂ (97 mg), benzyl ether (4 mL), Fe(CO)₅ (66 µL), oleyl amine (100 µL) and oleic acid (100 µL) were mixed under nitrogen. The reaction mixture was heated to 240 °C at heating rate of 15 °C/min. After 30 min, the heating source was removed and then the product was cooled to the room temperature. The particles were collected by centrifugation. After the aforementioned process, FePt nanoparticles with the lattice spacing of (111) were obtained.

The TEM image shows that the FePt nanoparticles of the present embodiment have particle sizes of 6.12±0.63 nm. In addition, the EDX analysis revealed that the composition of the FePt nanoparticles of the present embodiment is Fe₅₁Pt₄₉. Furthermore, the result of the magnetic measurement shows that the saturated mass magnetization (Mₛ) of the FePt nanoparticles of the present embodiment at 300 K is 3.2 emu/g Fe, as shown in FIG 2.

### Embodiment 3 - Preparation of 12-13 nm FePt nanoparticles

Pt(acac)₂ (195 mg), 1,2-hexadecandiol (1.05 g), dioctyl ether (4 mL), Fe(CO)₅ (66 µL), oleyl amine (4 mL) and oleic acid (4 mL) were mixed under N₂ and then heated to 240 °C at heating rate of 15 °C/min. The mixture was kept at 240 °C for 60 min. Then the heating source was removed and the reaction mixture was cooled to the room temperature. The final product was separated by addition of ethanol followed by centrifugation. After the aforementioned process, FePt nanoparticles with the lattice spacing of (100) were obtained.

The TEM image shows that the FePt nanoparticles of the present embodiment have particle sizes of 12.80±1.76 nm. In addition, the EDX analysis revealed that the composition of the FePt nanoparticles of the present embodiment is Fe₃₃Pt₆₇. Furthermore, the result of the magnetic measurement shows that the saturated mass magnetization (Mₛ) of the FePt nanoparticles of the present embodiment at 300 K is 12.3 emu/g Fe, as shown in FIG. 2.

### Embodiment 4 - Preparation of 3-4 nm CT/MRI dual-modality imaging contrast agent

Dry FePt nanoparticles of Embodiment 1 (100 mg) were dispersed in ethanol by sonication. Cysteinamine (∼1 g) was added and dissolved into this solution at room temperature. This mixture was sonicated at 40-50 °C overnight. Then the sample was washed to remove ligand unadsorbed on particles by ethanol. Finally, the modified particles were collected and stored in the bottle filled with N₂.

The FePt nanoparticles modified with amine groups were incubated with ethyl-3-[3-dimethylaminopropyl] carbodiimide hydrochloride (EDC) and mouse anti-Her2 antibodies. The solutions are stirred at 4 °C for 1 hr. The pallets were centrifuged in 13000 rpm for 15 min and then washed with phosphate-buffered saline (PBS) twice before next steps. After the aforementioned process, CT/MRI dual-modality targeting imaging contrast agents were obtained.

As shown in FIG. 1, the CT/MRI dual-modality targeting imaging contrast agent of the present embodiment comprises: an FePt nanoparticle 100; amino-group modification molecules 101 with mercapto groups (-SH) and amino groups (-NH₂), wherein the amino-group modification molecules 101 bind to the surface of the FePt nanoparticle 100 through the mercapto groups; and antibodies 102 with carboxyl groups (-COOH), wherein the antibodies 102 bind to the amino-group modification molecules 101 through bonds between the carboxyl groups and the amino groups. In addition, the FePt nanoparticle is in a form of sphere, and the composition thereof is Fe₅₈Pt₄₂.

### Embodiment 5 - Preparation of 6-7 nm CT/MRI dual-modality imaging contrast agent

The method for manufacturing the CT/MRI dual-modality imaging contrast agent of the present embodiment is the same as that described in Embodiment 4, except that the FePt nanoparticles prepared in Embodiment 2 is substituted for the FePt nanoparticles of Embodiment 1.

### Embodiment 6 - Preparation of 12-13 nm CT/MRI dual-modality imaging contrast agent

The method for manufacturing the CT/MRI dual-modality imaging contrast agent of the present embodiment is the same as that described in Embodiment 4, except that the FePt nanoparticles prepared in Embodiment 3 is substituted for the FePt nanoparticles of Embodiment 1.

### Evaluation of effects of FePt nanoparticles and dual-modality imaging contrast agents

### Cell culture

Human oral epidermoid carcinoma cell line (OECM1) was maintained in RPMI-1640 medium supplemented with 10% (v/v) fetal bovine serum (FBS). Vero cells were maintained in DMEM medium containing 10% (v/v) fetal bovine serum (FBS), 2 mM L-glutamine and 50 mg/mL gentamicin. The murine MBT-2 cell line derived from a carcinogen-induced bladder tumor in C3H/HeN mice was obtained from American type culture collection (ATCC, Manassas, VA). Cells were maintained in DMEM supplemented with 10% fetal bovine serum (FBS), 25 mM N-2-hydroxyethylpiperazine-N-2-ethanesulfonic acid (HEPES), 2 mM L-glutamine, and 1 mM sodium pyruvate. All the cell lines were maintained in a 37 °C incubator with a humidified environment of 5% CO2 in the air. Medium was changed every two days.

### In vitro cytotoxicity assay (MTT assay)

5 x 10³ Vero cells/well were seeded in 96-well plates with DMEM supplemented with 10% FBS, 2 mM L-glutamine and 50 mg/mL gentamicin. After 24 h, various sizes and serial 10-fold dilutions of the FePt nanoparticles (Embodiments 1-3) were added to cells with final concentrations ranging from 0.01 to 100 mM (iron cation concentration) for additional 24hr. Cell viability was detected using the 5-dimethylthiazol-2-yl-2, 5-diphenyl tetrazolium bromide (MTT) assay. After the cells were treated with MTT and incubated at 37 °C for 4 hr, the purple formazan in supernatant was quantified by measuring 595 nm absorbance by a spectrophotometer.

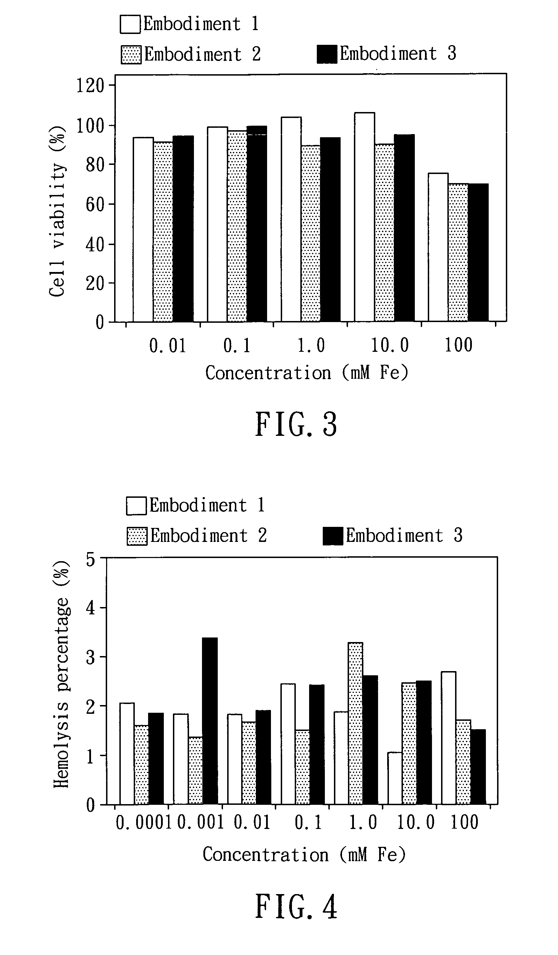

As shown in FIG. 3, the results of the MTT assay showed no significant cytotoxic response (the cell viability > 90%) detected at concentration below 10 mM after 24 hr of exposure of the FePt nanoparticles. Futher, at the highest concentration of the FePt nanoparticles (100 mM), the cell viability was still up to 75%.

### Hemolysis assay

Hemolysis assay was performed using human whole blood from a healthy donor with informed consent following IRB guidelines. The FePt nanoparticles of Embodiments 1-3 were added to the 200 µL human whole blood stored in the Vacutainer (BD Inc., USA) containing 24 IU sodium heparin, and the final concentrations of the FePt nanoparticles are in the range of 0.0001 to 100 mM (iron concentration). The tubes were gently mixed in a rotary shaker, and then incubated for 4 hr at 37 °C. The specimens were then centrifuged under 1200 rpm for 10 min to collect the serum. The serum was further centrifuged under 13000 rpm for 15 min to remove the FePt nanoparticles and the supernatant was analyzed for the presence of the hemoglobin by specific 545 nm spectrophotometric absorption.

As shown in FIG. 4, the results indicated that no significant hemolysis (< 5%) occurred in all FePt nanoparticles at various concentrations and particle sizes.

According to the results of MTT assay and hemolysis assay, high cell viability and low hemolysis for FePt nanoparticles with different particle sizes and suggest their excellent in vitro biocompatibility.

### Organ distribution of FePt nanoparticles

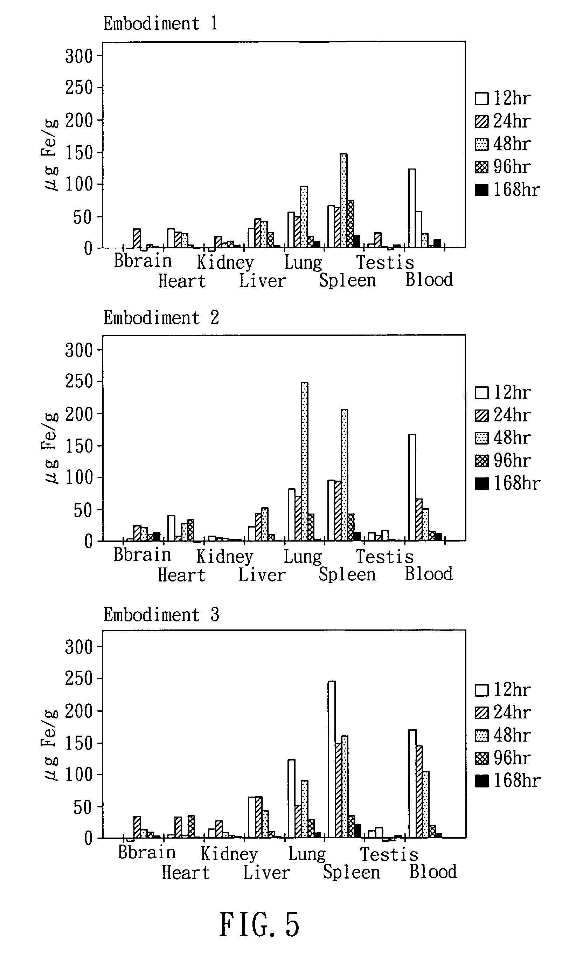

Six-week old male C3H/HeN mice purchased from animal center of NCKU were anesthetized by 40mg/Kg pantothol and were injected FePt nanoparticles of Embodiments 1-3 through tail vein at dose of 5mg/kg for 12, 24, 48, 96,168 hr before sacrificed. After saline perfusion, organs such as the brain, heart, lung, spleen, liver, kidney, testis and blood were collected and ground with nitro-hydrochloride acid. After filtered, the solution samples were analyzed by a flame atomic absorption spectrometer (UNICAM solar M6 series). The analytic results are shown in FIG. 5.

As shown in FIG. 5, most of the nanoparticles were cleared from the major organs after one week (168 hr). Larger particle size has higher serum concentration and longer serum half-life, and all three sizes of particles (i.e. FePt nanoparticles of Embodiments 1-3) approach the background levels after 96 hr. There are 102.2 µg/g of 12nm-FePt nanoparticles (Embodiment 3) in the serum 48 hr after injection, while only 22.2 µg/g of the 3nm-FePt nanoparticles (Embodiment 1) and 49.6 µg/g of the 6nm-FePt nanoparticles (Embodiment 2) were detected at the same time point. The FePt nanoparticles were mainly accumulated in the spleen followed by lung and liver and were gradually cleared from these organs with time. The 12nm-FePt nanoparticles (Embodiment 3) reached the plateau concentrations in these organs at 12 hr after injection, while it was 48 hr for the 3nm-FePt and 6nm-FePt nanoparticles (Embodiments 1 and 2). The highest tissue concentrations of the nanoparticles in the spleen and lung for 12nm-FePt nanoparticles (Embodiment 3) were 241.5 µg/g and 120.4 µg/g, while they were 204.9 µg/g and 247.9 µg/g for 6nm-FePt nanoparticles (Embodiment 2). The plateau concentrations of for 3nm-FePt nanoparticles (Embodiment 1) in the two organs was 146.6 µg/g in the spleen and 96.5 µg/g in the lung at 48hr. In addition, 6nm-FePt nanoparticles (Embodiment 2) presented the lowest non-specific hepatic uptake. Furthermore, the results also show that transient accumulation of the FePt nanoparticles in the brain in 24 hr for all FePt nanoparticles of Embodiments 1-3. The 3nm-FePt nanoparticles (Embodiment 1) has the highest brain concentration consistent with the reported size limit of the blood-brain-barrier.

Combined the biocompatibility (i.e. MTT assay), hemocompatibility (i.e. hemolysis assay) and the bio-distribution results, the FePt nanoparticles could potentially serve the *in vivo* applications.

### In vitro MRI and CT image

The FePt nanoparticles of Embodiments 1-3 at different concentration (0.01-100 mM) and the PBS control wee added into Eppendorf for MRI and CT contrast imaging examinations.

The magnetic resonance imaging (MRI) for *in vitro* study was performed by the following sequences: T₂-weighted three dimensional fast-field echo sequences (repetition time in ms/echo time in ms/flip angle/number of acquisitions/550/15/15°/3), field of view of 140×100 mm, a matrix of 256 × 196 pixels and a slice thickness of 1.4 mm.

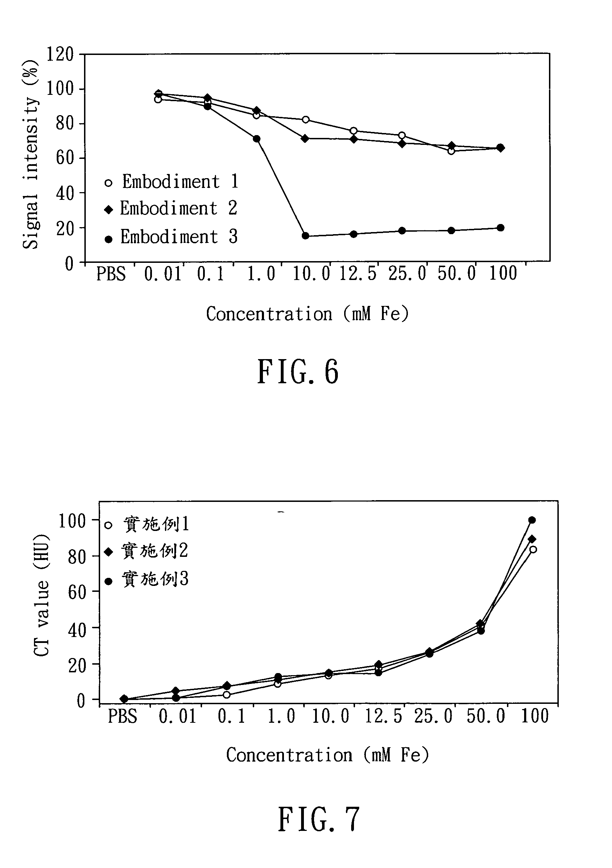

The results of the *in vitro* MRI show that a significant dose dependent inverse MR image contrast was observed in the FePt nanoparticles of Embodiments 1-3. The calculated intensity of each pellet was referred to that of PBS pellet and the normalized results were shown in FIG. 6. A shown in FIG. 6, the FePt nanoparticles of Embodiment 3 presented the most effective negative MR contrast and the signal intensity decreased by 86 % at the low concentration of 1 mM Fe. In addition, the results also showed detectably image darkening by about 28 % for 3nm-FePt nanoparticles of Embodiment 1 and about 33 % for 6nm-FePt nanoparticles of Embodiment 2 at 25 mM Fe. Furthermore, 12nm-FePt nanoparticles of Embodiment 3 exerted the best inverse contrast effect in the in vitro experiment in a dose dependent manner.

The computed tomography (CT) data were acquired using a GE Light Speed VCT 64-detector CT. Imaging parameters were as follows: slice thickness, 0.625 mm; 120 kVp, 30 mA; field of view, 512 x 512, gantry rotation time, 0.4 s; table speed, 40 mm/rotation.

The results of the *in vitro* CT imaging show that positive contrast enhancement in a dose dependent manner was observed in the FePt nanoparticles of Embodiments 1-3, and appreciable enhancement for CT contrast was especially observed at 1 mM Fe for all FePt nanoparticles of Embodiments 1-3. The calculated CT signal contrast intensities within the range of 0.01 to 100 mM Fe were shown in FIG. 7. A shown in FIG. 7, the CT signal of 12nm-FePt nanoparticles of Embodiment 3 at 100 mM Fe (corresponding to 44.7 mg FePt/mL) exhibited the equivalent ability of CT contrast as 48.4 mg/mL of the popular iodine-containing agent. In other words, the contrast effect of 12nm-FePt nanoparticles of Embodiment 3 was about equal to that of the current iodine-containing agent at the same concentration. In addition, the contrast effects of 3nm-FePt nanoparticles of Embodiment 1 and 6nm-FePt nanoparticles of Embodiment 2 were approximately 2-fold higher than that of current iodine-containing agent at the same concentration.

According to these results, FePt nanoparticles of Embodiments 1-3 enhanced shortening T₂ of the proton relaxation due to their superparamagnetic property, while the high X-ray absorption coefficient (4.99 cm²/g for Pt component) thereof enable their efficient CT contrast enhancement. In addition, the size-dependent relationship in mass magnetization and X-ray absorption reflected the results of the *in vitro* MR imaging and CT contrast. Thus, the FePt nanoparticles of Embodiments 1-3 have great potential to serve as CT and MRI dual-modality contrast imaging agents.

### In vitro MRI and CT image of CTlMRI dual-modality targeting imaging contrast agents

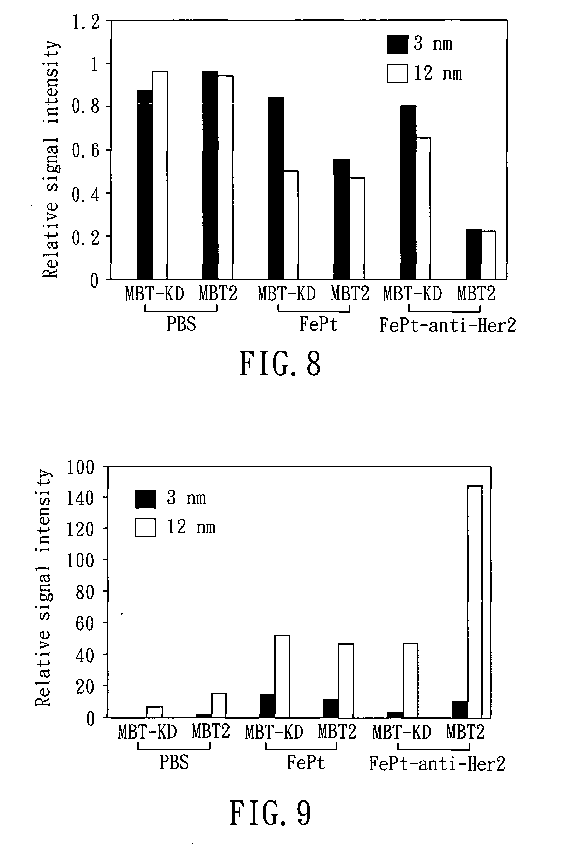

MBT2 cell with Her2/neu knock down (MBT-KD) and the wild type cell (MBT2) were used as the cell models. 10⁷ cells were fixed by 4% paraformaldehyde, resuspended into a 15 ml tube, and then incubated with anti-Her2 antibody tagged FePt nanoparticles (i.e. CT/MRI dual-modality targeting imaging contrast agents of Embodiments 4 and 6) and the control nanoparticles (i.e. FePt nanoparticles of Embodiments 1 and 3) in final iron concentration of 1 mM. After 4hr incubation, 10⁷ cells in each group were placed in an Eppendorf tube array and analyzed for the contrast effect in an MR imager. The following pulse sequences were used: T₂-weighted sequence: fast spin echo with TR=550, TE=15, echo train length (ET) =10 ms. The array was then subject to CT scan following the aforementioned parameters. The MR signal intensity of FePt nanoparticles normalized by that of the cell pellets in PBS solution was shown in FIG. 8, and the CT signal intensity of the pellets of FePt nanoparticles and that of the cell pellets in PBS solution was shown in FIG. 9.

As shown in FIG. 8, the signal intensities of the cell pellets showed minor signal reduction (3.6%) for the MBT-KD while a 42% reduction in MR signal was observed in the MBT2 when 3 nm-FePt nanoparticles of Embodiment 1 were applied. These results suggest that modulation of Her2/neu expression may have certain effect to the non-specific uptake activity of 3nm-FePt nanoparticles of Embodiment 1. However, both MBT-KD and MBT2 cells showed about equal signal intensity reduction when targeted by 12nm-FePt nanoparticles of Embodiment 3 (47.6% for MBT-KD and 49.7% for MBT2). These results suggest that non-specific uptake of 12nm-FePt nanoparticles of Embodiment 3 is not significantly affected by the Her2/neu expression. In contrast, both dual-modality targeting imaging contrast agent of Embodiments 4 and 6 (i.e. 3nm-FePt-anti-Her2) and 12nm-FePt-anti-Her2 presented a significant Her2/neu expression dependent signal reduction. A drastic reduction in MRI signal intensity (73.7% for 3nm-FePt-anti-Her2 and 65.0% for 12nm-FePt-anti-Her2) was observed when compared the MRI signal of the MBT-KD to that of the Her-2 knock down line MBT-KD. Hence, the cysteinamine ligand on these FePt nanoparticles can enhance the effect of cellar uptake and endocytosis. In addition, the appreciable differences in the targeting nanoparticles conjugated with anti-Her2 antibody demonstrated the selectivity for Her2/neu high expression cells. Most worthily, dual-modality targeting imaging contrast agent of Embodiment 6 exerted the best MR contrast due to their higher magnetic susceptibility.

As shown in FIG. 9, a 3.3 times positive contrast enhancement was observed in MBT2 compared to MBT-KD. However, the overall CT intensity was low in all test samples compared to 12nm particles. CT contrast enhancement was observed in both MBT-KD (7.7X) and MBT2 (3.1X) targeted by 12nm-FePt nanoparticles. In addition, the 12nm-FePt-anti-Her2 successfully differentiates Her2-overexpress wild type cells (MBT2) to the gene knock down line (MBT-KD), and a 3.1X signal intensity enhancement was detected.

These results suggest the great potential for the 12nm-FePt-anti-Her2 for dual-modality targeting imaging contrast agents in MRI (inverse contrast) and CT (positive contrast).

### In vivo MRI and CT image of CT/MRI dual-modality targeting imaging contrast agents

The in vivo molecular imaging was performed in mice bearing transplanted MBT2 tumor. The male C3H/HeN mice 6- to 8 weeks old was provided form National Cheng Kung University Laboratory Animal Center (Tainan, Taiwan). MBT-2 tumor lesions were established by subcutaneous dorsal flank injection of 10⁷ tumor cells in 100µL normal saline using a 27-gauge needle. Visible tumor was normally observed one week after implantation. Animals were anesthetized using 2% isoflurane (Abbott Laboratories, Abbott Park, IL) mixed with 100% O₂, which is delivered with a veterinary anesthesia delivery system (ADS 1000; Engler Engineering Corp., Hialeah, FL). The tumor lesions were then subject to the two imaging modality analysis after tail vein injection of dual-modality targeting imaging contrast agents of Embodiments 4 and 6 (5 mg Fe/kg). The images were taken at a time sequence from 0-24hrs using T₂-weighted MR acquisition sequence with the following parameters: fast spin echo with TR/TE=3000 ms/99.7 ms, ET=10 ms. The signal intensity of tissue in each test was determined by standard region-of-interest measurements of cross-sectional image of the tissue using the provided image quantification software.

The *in vivo* micro-computed tomography analysis (Skyscan 1076 X-ray Microtomograph, Skyscan, Aartselaar, Belgium) was then performed using micro-focused x-ray source (50keV /200Ua) illumination, and each image acquisition was performed when rotated one step (1 degree) through 360 degrees. The images were processed for cross sections by reconstruction using NRecon (Skyscan) software.

The results of the MR images show that a significant reduction of tumor lesion intensity to 51% was observed at 24 hr after injection, with respects to the MR images at time 0 hr. On the other hand, a significant 138% contrast enhancement of the tumor tissue was observed at 24hr in CT image analysis.

These results of the *in vivo* experiments shows that the dual-modality targeting imaging contrast agents of Embodiments 4-6 can selectively target tumor lesions, and increase the contrast of T₂ MRI sequence and CT modalities. Especially, the 12 nm dual-modality targeting imaging contrast agents of Embodiment 6 presents great potential as the dual-modality targeting imaging contrast agents for CT and MRI examinations.

Although the present invention has been explained in relation to its preferred embodiment, it is to be understood that many other possible modifications and variations can be made without departing from the spirit and scope of the invention as hereinafter claimed.

## Claims

1. A method for manufacturing a CT/MRI dual-modality imaging contrast agent, comprising the following steps:
(A) providing a mixture solution containing a Pt compound, a Fe compound, a polyol, a surfactant, and a solvent;
(B) heating the mixture solution to react the Pt compound with the Fe compound to obtain FePt nanoparticles;
(C) cooling the mixture solution, and separating the FePt nanoparticles from the mixture solution; and
(D) mixing the FePt nanoparticles with a surface modification molecule to obtain surface-modified FePt nanoparticles.

2. The method as claimed in claim 1, further comprising a step (E) after the step (D): mixing the surface-modified FePt nanoparticles with target molecules to obtain a dual-modality targeting imaging contrast agent.

3. The method as claimed in claim 1, wherein the Pt compound is at least one selected from the group consisting of Pt(acac)₂, PtCl₂, PtCl₄, H₂PtCl₄, H₂PtCl₆, K₂PtCl₄, and K₂PtCl₆.

4. The method as claimed in claim 1, wherein the Fe compound is at least one selected from the group consisting of Fe(CO)₅, Fe(acac)₂, Fe(acac)₃, and Fe-oleate.

5. The method as claimed in claim 1, wherein the surfacant is at least one selected from the group consisting of oleic acid, and oleyl amine.

6. The method as claimed in claim 1, wherein the surfactant comprises oleic acid, and oleyl amine.

7. The method as claimed in claim 5, wherein the volume ratio between the oleic acid and the oleyl amine is 1:1 to 3:1.

8. The method as claimed in claim 1, wherein the polyol is C₈₋₂₀ alcohol.

9. The method as claimed in claim 1, wherein the polyol is at least one selected from the group consisting of 1,2-hexa-decanediol, 1,2-Dodecanediol, and polyethylene glycol.

10. The method as claimed in claim 1, wherein the surface modification molecule is cysteinamine, cystamine, or mercaptoalkylamine.

11. The method as claimed in claim 1, wherein the target molecules are Her2 antibodies.

12. The method as claimed in claim 1, wherein the FePt nanoparticles are represented by the following formula (I):
FeₓPt₁₀₀₋ₓ (I)
wherein, x is 30-60.

13. The method as claimed in claim 1, wherein the diameter of the FePt nanoparticles is 1-20 nm.

14. The method as claimed in claim 1, wherein the FePt nanoparticles are in a form of sphere, octapod, cuboctahedron, cube, or truncated cube.

15. A CT/MRI dual-modality imaging contrast agent, comprising:
an FePt nanoparticle; and
a surface modification molecule binding to a surface of the FePt nanoparticle, wherein the surface modification molecule contains an amino-group, a carboxyl-group, or a biotin,
wherein, the FePt nanoparticle is in a form of sphere, octapod, cuboctahedron, cube, or truncated cube, and the FePt nanoparticle is represented by the following formula (I):
FeₓPt₁₀₀₋ₓ (I)
wherein, x is 30-60.

16. The CT/MRI dual-modality imaging contrast agent as claimed in claim 15, further comprising a target molecule binding to the surface modification molecule, wherein the target molecule is an antibody, a protein, or nucleic acids.

17. The CT/MRI dual-modality imaging contrast agent as claimed in claim 15, wherein the surface modification molecule is an amino-group modification molecule with a mercapto group (-SH), and an amino group (-NH₂), the amino-group modification molecule binds to the surface of the FePt nanoparticle through the mercapto group, and the surface of the FePt nanoparticle is modified with the amino group.

18. The CT/MRI dual-modality imaging contrast agent as claimed in claim 17, further comprising a target molecule containing a carboxyl group (-COOH), wherein the target molecule binds to the amino-group modification molecule through the bonding between the amino group and the carboxyl group.

19. The CT/MRI dual-modality imaging contrast agent as claimed in claim 15, wherein the surface modification molecule is cysteinamine, cystamine, or mercaptoalkylamine.

20. The CT/MRI dual-modality imaging contrast agent as claimed in claim 15, wherein the diameter of the FePt nanoparticles is 1-20 nm.

21. The CT/MRI dual-modality imaging contrast agent as claimed in claim 16, wherein the antibody is a Her2 antibody.
